# EUROPÄISCHE PATENTSCHRIFT

(11) **EP 1 900 388 B1**
(45) Veröffentlichungstag und Bekanntmachung des Hinweises auf die Patenterteilung: **05.06.2013**
(21) Anmeldenummer: 07022977.8
(22) Anmeldetag: 25.02.2000
(51) Int. Cl.: A61M 16/06

(54) **Dichtlippeneinrichtung für eine Atemmaske**
Seal device for a respiratory mask
Dispositif à lèvre d'étanchéité pour un masque respiratoire

(43) Veröffentlichungstag der Anmeldung: 19.03.2008
(62) Teilanmeldung aus: 00912509.7
(73) Patentinhaber: ResMed R&D Germany GmbH, 82152 Martinsried (DE)
(72) Erfinder: Melidis, Paris, 82140 Olching (DE); Lauboeck, Theodor, 85662 Hohenbrunn (DE)
(74) Vertreter: Vossius & Partner

(56) Entgegenhaltungen:
- EP-A- 0 747 078
- WO-A-98/04310
- DE-A- 4 233 448
- US-A- 2 133 699
- US-A- 2 706 983
- US-A- 4 907 584

## Beschreibung

Die Erfindung betrifft eine Dichtlippeneindchtung für eine Atemmaske und eine Atemmaske an sich.

Insbesondere betrifft die Erfindung auf Atemmasken welche in abdichtender Weise auf den Nasenbereich aufsetzbar sind und hierbei eine im Bereich der Oberlippe des Maskenanwenders zwischen Mund und Nase verlaufende Abdichtungseinrichtung aufweisen. Atemmasken finden insbesondere im medizinischen sowie im technischen und Bereich zur Zufuhr eines Atemgases insbesondere unter Überdruck, Anwendung.

Bei diesen Atemmasken wird eine Abdichtung zur Gesichtsfläche eines Anwenders üblicherweise durch eine umlaufende und aus einem elastomeren Material gefertigte Dichtlippe erreicht.

Die mit einer derartigen Dichtlippe erreichte Dichtwirkung nimmt allgemein mit dem Anpreßdruck der Dichtlippe gegen die Gesichtsfläche zu. Durch vergleichsweise hohe Anpreßdrücke wird jedoch der Tragekomfort beeinträchtigt. Je nach Empfindlichkeit des Maskenanwenders bereitet die Langzeitanwendung der bekannten Atemmasken Unannehmlichkeiten.

Dokument US 2,706,983 offenbart eine Atemmaske mit einem Gesichtstück, welches eine Stirnwand und einen nach innen gerichteten und das Gesicht berührenden Randbereich aufweist. Ferner weist die Atemmaske eine Mehrzahl von Faltenbereichen auf.

Der Erfindung liegt die Aufgabe zugrunde, eine Atemmaske zu schaffen bei welcher eine hohe Dichtwirkung auf zuverlässige Weise unter einem hohen Tragekomfort erreicht werden kann.

Diese Aufgabe wird erfindungsgemäß gelöst durch den Gegenstand des Patenanspruchs 1, wobei eine Dichtlippeneinrichtung für eine Atemmaske unter anderem eine Aufnahmeöffnung zur Aufnahme wenigstens des Nasen-spitzenbereiches eines Maskenanwenders und eine aus einem elastomeren Material gebildeten, um die Aufnahmeöffnung umlaufenden und in Applikationsposition den Nasenrücken überquerenden Dichtlippe aufweist. Die Dichtlippe weist eine zur Auflage auf dem Gesicht eines Maskenanwenders vorgesehene Auflagezone auf, wobei die Dichtlippe derart elastisch nachgiebig angeordnet ist, daß sich im Bereich des Nasenrückens eine höhere Nachgiebigkeit ergibt als im Bereich der Nasenflügel und/oder der Oberlippe.

Dadurch wird in vorteilhafter Weise bei einem hohen Tragekomfort eine hohe Kompatibilität zu unterschiedlichsten Gesichtstekturen erreicht. Die erfindungsgemäße Atemmaske zeichnet sich insbesondere im Bereich des Nasenrückens durch eine hohe Dichtigkeit aus, ohne daß hierbei erhebliche Flächenpressungen auftreten. Durch die im Bereich des Nasenrückens erreichte hohe Dichtwirkung wird insbesondere Augenreizungen und Zugluftwahrnehmungen wirkungsvoll vorgebeugt.

Die definierte Nachgiebigkeit der auf dem Nasenrückenbereich aufsitzenden Dichtlippenzone wird dadurch erreicht, daß die Dichtlippeneinrichtung im Bereich dieser Zone an einer Faltenbalgstruktur aufgehängt ist.

Diese Faltenbalgstruktur ist vorzugsweise derart dimensioniert, daß diese bei entsprechend tiefem Eindringen des Nasenrückens, eine Anschlageinrichtung bildet Die Hierbei zur Wirkung kommenden Anschlagflächen sind vorzugsweise derart ausgebildet, daß diese spätestens im eingefederten Zustand eine vergleichsweise großflächige Auflagerfläche bilden, so daß sich selbst bei Wirksamwerden der Faltenbalgstruktur im eingefederten Zustand keine unzulässig hohen Flächenpressungen ergeben.

In besonders vorteilhafter Weise ist der Faltenbalgstruktur eine durch unterschiedliche Wandstärken definierte Gelenkcharakteristik verliehen. Vorzugsweise sind die Knick- oder Gelenkstelle vergleichsweise dünnwandig ausgebildet, wogegen die dazwischen liegenden Zonen geringfügig dicker ausgebildet sind. Alternativ hierzu oder auch in Kombination mit dieser Maßnahme ist es auch möglich, Rollbalgstrukturen durch entsprechende Wanddicken vorzusehen.

In besonders vorteilhafter Weise weist die Faltenbalgstruktur mehrere Falteneinzüge auf. Vorzugsweise erstreckt sich wenigstens ein Falteneinzug vom Nasenrückenbereich bis in einen, in Gebrauchsposition der Maske den Nasenflügeln benachbarten Bereich hinein.

Insbesondere bei mehreren Falteneinzügen erstreckt sich vorzugsweise wenigstens einer derselben um den gesamten Umfang der Dichtlippeneinrichtung herum. Die Federcharakteristik des jeweiligen Falteneinzuges kann für bestimmte Umfangszonen derart definiert festgelegt werden, daß sich im Nasenrückenbereich eine höhere Nachgiebigkeit und im Bereich der Oberlippe oder insbesondere im Bereich der Nasenflügel (Diese Angaben erfolgen unter Bezugnahme auf die Applikationsposition der Maske) eine geringere Nachgiebigkeit ergibt.

Insbesondere unter Anwendung der Faltenbalgstruktur im Nasenrückendichtzonenbereich ist die Dichtungseinrichtung vorzugsweise derart ausgebildet, daß die, entgegen der Applikationsrichtung erreichte Nachgiebigkeit der Dichtlippe derart abgestimmt ist, daß sich im Nasenflügel- oder Oberlippenbereich eine Adaptions- bzw. Artikulationsachse ergibt. Hierdurch wird es möglich, die entsprechende Atemmaske auf dem Gesicht des Maskenanwenders überwiegend im Bereich der den Nasenflügeln benachbarten Gesichtszonen sowie auf der Oberlippe aufzusetzen wobei die vorzugsweise äußerst dünnwandig ausgebildete, zur Abdichtung des Nasenrückens vorgesehene Dichtlippenzone entsprechend der Gesichtstektur gegenüber dem Maskenrahmen verschwenkt werden kann. Durch den in der Maske herrschenden Innendruck kann dann dies schwenkbar gelagerte Dichtlippenzone gleichmäßig auf den Nasenrücken des Maskenanwenders aufgepreßt werden ohne daß hierbei den Innendruck der Maske erheblich übersteigende Flächenpressungen eintreten.

Die besonders vorteilhafte Kinematik und Gelenkcharakteristik des durch die Dichtlippeneinrichtung gebildeten Maskenkissens kann insbesondere dadurch erreicht werden, daß in dem, den Nasenflügeln oder der Oberlippe benachbarten Bereich der Dichtlippe lokal Zonen höherer Tragfähigkeit ausgebildet sind.

Die Zonen höherer Tragfähigkeit sind gemäß einer besonders bevorzugten Ausführungsform der Erfindung durch lokal verdickte Zonen der Dichtlippe gebildet. Der Übergang der lokal verdickten Zonen erfolgt vorzugsweise entlang linsenrandartiger Bereiche oder auch in flach auslaufender Weise ggf. ohne daß der Übergang zwischen den Zonen deutlich erkennbar ist.

Gemäß einer besonders bevorzugten Ausführungsform der Erfindung stützen sich die lokal verdickten Zonen über eine in die Dichtlippe eingeformte Stützstruktur auf einer Maskenrahmenzone ab. Diese Maskenrahmenzone ist vorzugsweise dickwandig ausgebildet und weist hierbei eine Wandstärke im Bereich von 3 bis 6 mm auf.

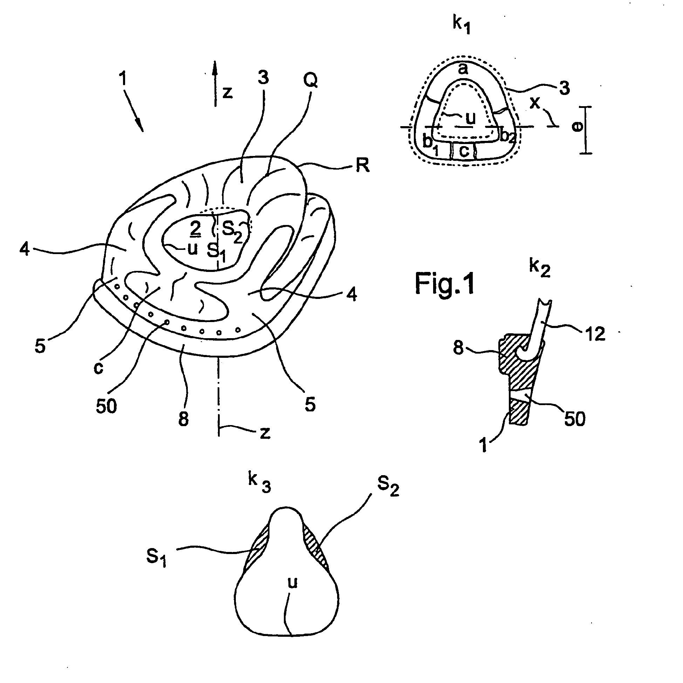

Die Zonen höherer Tragfähigkeit sind vorzugsweise pad-artig ausgebildet wie dies beispielhaft in Figur 1 dargestellt ist auf welche später noch ausführlich Bezug genommen werden wird.

Eine unter ergonomischen Gesichtspunkten besonders vorteilhafte Abstützung des Maskenkissens wird dadurch erreicht, daß die Zonen höherer Tragfähigkeit im Bereich der Gesichtskontaktzone jeweils eine im wesentlichen mondsichelförmige Gestalt aufweisen. Die im Bereich der zur Auflage auf der Oberlippe vorgesehenen Schenkel jener Zonen höherer Tragfähigkeit sind vorzugsweise derart verkürzt ausgebildet, daß im Bereich der Oberlippe in einem zwischen den Zonen höherer Tragfähigkeit eine Zone hoher Elastizität und Nachgiebigkeit entgegen der Applikationsrichtung ausgebildet ist. Diese höhere Nachgiebigkeit kann in vorteilhafter Weise erreicht werden indem hier ebenfalls eine lokale Faltenstruktur oder eine entsprechend dünnwandige Zone vorgesehen ist.

Die Dichtlippeneinrichtung ist gemäß einer besonders bevorzugten Ausführungsform der Erfindung an einem Maskenbasiskörper angebracht. Der Maskenbasiskörper kann ebenfalls aus einem elastomeren Material beispielsweise Silikonkautschuk gebildet sein. Gemäß einer besonders bevorzugten Ausführungsform der Erfindung ist der Maskenbasiskörper jedoch durch eine Hartschale beispielsweise aus einem volltransparenten Material, gebildet. Diese Hartschale weist vorzugsweise einen, in Applikationsposition zum Stirnbereich des Maskenanwenders weisenden Leitungsanschluß auf. Alternativ hierzu ist es auch möglich, die Hartschale mit einer zentralen oder seitlichen Anschlußstuktur zur Ankoppelung einer Atemgasleitung zu versehen.

Die Anbringung der Dichtlippeneinrichtung bzw. des Dichtkissens an der Hartschale oder einem Maskenbasiskörper erfolgt vorzugsweise durch Verwendung einer Koppelungsstruktur. Diese Koppelungsstruktur weist gemäß einer besonders bevorzugten Ausführungsform der Erfindung seitens der Hartschale einen Umfangswulstabschnitt und seitens der Dichtlippeneinrichtung einen Rahmenabschnitt mit einem komplementären Aufnahmefalz auf. Falz und Wulst sind vorzugsweise derart ausgebildet, daß sich im Falle einer Maskeninnendruck bedingten Aufweitung des Maskenkissens im Bereich der Koppelungsstruktur Flächenpressungen ergeben die stets höher sind als der Maskeninnendruck. Hierdurch wird eine besonders zuverlässige Dichtwirkung ohne Zusatz von Klebstoffen erreicht.

In besonders vorteilhafter Weise sind Mittel vorgesehen zur Fixierung der Position der Dichtlippeneinrichtung gegenüber der Hartschale in Umfangsrichtung. Diese Mittel können beispielsweise durch Positioniervorsprünge oder insbesondere durch Durchbrechungen des Umfangswulstes gebildet sein.

Der Dichtlippeneinrichtung ist vorzugsweise eine Vorspannung verliehen die in vorteilhafter Weise durch elastische Verformung bei der Koppelung mit der Hartschale erreicht wird. Hierdurch wird es möglich das Verformungsverhalten der Dichtlippeneinrichtung definiert zu beeinflussen. Insbesondere ist es möglich bestimmte Zonen der Dichtlippeneinrichtung derart vorzuspannen, das der Bildung von Kräuselfalten im Bereich der Gesichtsabdichtungszone auf vorteilhafte Weise vorgebeugt ist.

Der Rahmenabschnitt ist gemäß einer vorteilhaften Ausführungsform der Erfindung derart ausgebildet, daß dieser im wesentlichen in einer Ebene verläuft. Hierdurch wird eine vergleichsweise flache Bauweise der Hartschale und einfache Vorspannung des Maskenkissens möglich.

Alternativ hierzu ist es jedoch auch möglich, die Maskenanordnung derart auszubilden, daß der Rahmenabschnitt einen im Bereich der Artikulationsachse zur Zone hoher Tragfähigkeit vordringenden Verlauf aufweist. Hierdurch wird es möglich bereits der Hartschale selbst einen weitgehend der statistisch wahrscheinlichsten Gesichtstektur entsprechenden Verlauf zu verleihen.

In vorteilhafter Weise liegt die Wanddicke der dünnen Zone im Bereich von 0,65 bis 1,85 mm. Diese Wanddicke verleiht der Maske eine auch bei Maskendrücken im Bereich von 15 mbar ausreichende Druckfestigkeit.

Die Wanddicke der Zone hoher Tragfähigkeit liegt vorzugsweise im Bereich von 0,80 bis 4mm.

Besonders bevorzugt wird das Maskenkissen durch ein mehrstufiges Formraumfüllverfahren hergestellt. Hierdurch wird es möglich, der Zone hoher Tragfähigkeit eine gegenüber der Zone geringer Tragfähigkeit abweichende Färbung zu verleihen. Auch ist es möglich die mechanischen Eigenschaften der für die jeweilige Zone jeweils verwendeten Werkstoffe definiert abzustimmen.

Die Zone hoher Tragfähigkeit ist vorzugsweise durch zwei vom unteren Eckbereich des Rahmenabschnittes aufragende und in die Dichtlippe als flache Schenkel ausfließende elastomere Abschnitte gebildet. Die Dichtlippe selbst ist vorzugsweise aus einem elastomeren Material insbesondere volltransparentern Silikonkautschuk gebildet Die unmittelbar mit dem Gesicht des Maskenanwenders in Kontakt tretende Außenfläche des Maskenkissens ist vorzugsweise samtartig mattiert ausgebildet. Hierdurch wird ein verbessertes Tragegefühl erreicht

Eine unter fertigungstechnischen Gesichtspunkten besonders vorteilhafte Ausführungsform der Erfindung ist dadurch gegeben, daß die Hartschale an die Dichtlippeneinrichtung angespritzt ist Hierdurch wird neben einer besonders zuverlässigen Koppelung von Hartschale bzw. Maskenbasiskörper und dem Maskenkissen auch einer unter bakteriologischen Gesichtspunkten unvorteilhaften Spaltbildung vorgebeugt.

Bei einem bevorzugten Verfahren zur Herstellung einer erfindungsgemäßen Dichtlippeneinrichtung für eine Atemmaske wird ein elastomeres Material in einen durch ein Formwerkzeug gebildeten Formraum eingebracht, welches in dem Formraum zumindest teilweise abbindet und nach Öffnen des Formwerkzeuges aus diesem entnommen wird, wobei das elastomere Material in zwei zeitlich abfolgenden Schritten in den entsprechenden Formraum eingebracht wird.

Dadurch wird es möglich, ein Maskenkissen zu schaffen das eine einzige Dichtlippe aufweist welche eine nach Maßgabe der Belastbarkeit und statistisch erwarteten Tekturvarianz der entsprechenden Gesichtszone definierte Nachgiebigkeit in Applikationsrichtung aufweist.

In vorteilhafter Weise werden eine Trägerstruktur der Dichtlippeneinrichtung und eine dünnwandige Zone der Dichtlippe in zeitlich separaten Schritten und ggf. unter Verwendung von Materialien unterschiedlicher mechanischer Eigenschaften und ggf. Farbe, gebildet.

Vorzugsweise wird die Trägerstruktur in einem ersten Spritzschritt und die dünnwandige Zone in einem nachfolgenden zweiten Spritzschritt gebildet. Die Einbringung des jeweiligen Materials erfolgt vorzugsweise durch Spritzen oder vorab durch entsprechendes Einfüllen in den Formraum.

Der zur Füllung mit dem, die dünnwandige Zone bildenden Material vorgesehene Formraum wird vorzugsweise definiert, indem ein, eine Dichtlippenaußenseite begrenzendes Formwerkzeug von einem eine Dichtlippeninnenseite begrenzenden Kern abgehoben wird.

Alternativ hierzu ist es auch möglich, die Trägerstruktur durch einen Formraum zu bilden der durch einen, eine Dichtlippeninnenseite begrenzenden Kern und ein Aussenwerkzeug definiert ist, wobei zur Bildung der dünnwandigen Zone der Dichtlippe das Außenwerkzeug gewechselt wird und anschließend das zur Bildung der dünnen Zone vorgesehene Material in den nunmehr vorhandenen, für die dünnwandige Zone vorgesehenen Formraum eingebracht wird und hierin abbindet

In werkzeugtechnischer Hinsicht wird die eingangs angegebene Aufgabe gelöst durch ein Formwerkzeug zur Herstellung einer Dichtlippeneinrichtung für eine Atemmaske, mit einer Formkerneinrichtung die im Zusammenspiel mit einem Formaußenwerkzeug einen Formraum mit einem Faltenabschnitt definiert.

Dadurch wird es auf vorteilhafte Weise möglich, die Dichtlippeneinrichtung beispielsweise im Rahmen eines voll automatisierten Silikonspritzverfahrens herzustellen.

Das Formaußenwerkzeug ist bevorzugt mehrteilig ausgebildet. Vorzugsweise besteht das Außenwerkzeug aus einer die Dichtlippenaußenfläche begrenzenden Formhälfte und einer mit dieser zusammenwirkenden, den übrigen Bereich der Außenfläche des Dichtkissens begrenzenden Formhälfte. Der Innenbereich des Dichtkissens wird durch eine vorzugsweise einstückige Kemeinrichtung begrenzt. Bei der beschriebenen zweiteiligen Ausführungsform des Außenwerkzeuges ist es möglich, eine Außenwerkzeughälfte entlang einer Entformurigsachse anzuziehen, welche in eine zur Nasenrückenseite bzw. in eine der Oberlippendichtzone abgewandte Richtung verläuft. Die lokal im Nasenrückenbereich ausgebildete Faltenbalgzone und die Entformungsachse sowie der Verlauf des Koppelungsrahmens des Maskenkissens sind vorzugsweise derart abgestimmt, daß sich Entformungswinkel wenigstens im Bereich von 2° ergeben.

Die dem Maskenanwender zugewandte Dichtlippenaußenseite ist insbesondere hierbei vorzugsweise durch einen Außenformwerkzeugabschnitt im Zusammenspiel mit der Formkerneinrichtung gebildet, wobei der Außenformwerkzeugabschnitt eine umlaufende Formraumrinne aufweist welche die Dichtlippenaußenseite definiert.

Die äußere Trennkante der Formraumrinne erstreckt sich vorzugsweise im Bereich der äußeren Umfangskante der Dichtlippe. Hierdurch werden etwaige Grate im Bereich der Gesichtskontaktflächen auf vorteilhafte Weise vermieden.

Eine unter fertigungstechnischen Gesichtspunkten besonders vorteilhaft realisierbare Ausführungsform einer Leckageeinrichtung zur Ableitung von zumindest teilweise verbrauchter Atemluft in die Umgebung ist gem. einem besonderen Aspekt der Erfindung gegeben durch eine Atemmaske mit einem Maskenkörper und einer Dichtkisseneinrichtung, die aus einem elastomeren Material gebildet ist und im Zusammenspiel mit dem Maskenkörper einen Maskeninnenraum begrenzt und einer Auslaßeinrichtung zur Ableitung von zumindest teilweise verbrauchtem Atemgas aus dem Maskeninnenraum, wobei die Auslaßeinrichtung einen Strömungswegabschnitt aufweist, der zumindest teilweise durch die Dichtkisseneinrichtung definiert ist.

Diese Maßnahme kann auch unabhängig von den vorangehend beschriebenen Ausgestaltungen Anwendung finden. Vorteilhafte Weiterbildungen dieses, an sich eigenständigen, Erfindungskomplexes sind in den Unteransprüchen angegeben.

Weitere vorteilhafte Ausgestaltungen der Erfindung sind in den Unteransprüchen angegeben.

Weitere Einzelheiten der Erfindung ergeben sich aus der nachfolgenden Beschreibung in Verbindung mit der Zeichnung. Es zeigen:
**Fig.1** eine perspektivische Ansicht eines Maskenkissens gemäß einer ersten Ausführungsform der Erfindung mit einer lokalen Faltenbalgstruktur und pad-artigen Zonen erhöhter Tragfähigkeit im Bereich der in Applikationsposition den Nasenflügeln benachbarten Dichtlippen nebst zugehörigen Skizzen A1, A2 und A3 zur Illustration und begleitenden Erläuterung;
**Fig. 2** eine vereinfachte Seitenansicht einer weiteren Atemmaske mit einem Falteneinzug und einem im wesentlichen in einer Ebene verlaufenden Rahmenabschnitt;
**Fig. 3a** eine vereinfachte Seitenansicht einer weiteren Ausführungsform eines Maskenkissens ebenfalls mit einem balgartigen Falteneinzug und angedeuteter Adaptions- bzw. Artikulationsachse;
**Fig. 3b** eine vereinfachte Seitenansicht einer weiteren Ausführungsform einer Atemmaske mit einer nur im hinteren Drittel des Maskenkissens vorgesehenen Falte;
**Fig. 4** eine vereinfachte Draufsicht auf eine Dichtlippe sowie Diagrammen zur qualitativen Illustration einer bevorzugten Abstimmung der Tragfähigkeit der Dichtlippe;
**Fig. 5** eine Schnittansicht zur Erläuterung einer bevorzugten Ausführungsform eines Faltenbereiches mit einer durch Zonen unterschiedlicher Wanddicke festgelegten Gelenkcharakteristik einschließlich Prinzipskizze;
**Fig. 6** eine Skizze zur Erläuterung bevorzugter Querschnittsgestaltungen bei einer Dichtlippeneinrichtung gemäß der Erfindung;
**Fig. 7** eine Skizze zur Erläuterung der Nachgiebigkeit der gelenkartigen Aufhängung einer Dichtlippe;
**Fig. 8** eine Skizze zur Erläuterung einer bevorzugten Maßnahme zur Fixierung des Maskenkissens in Umfangsrichtung;
**Fig. 9** eine weitere Skizze zur Erläuterung eines bevorzugten Aufbaues eines Formwerkzeuges in Verbindung mit vorteilhaften Ausgestaltungen eines Maskenbasiskörpers (Hartschale);
**Fig. 10** eine vereinfachte Schnittansicht zur Erläuterung eines Faltenabschnittes mit mehreren Falteneinzügen und definierter Gelenkcharakteristik;
**Fig. 11** eine perspektivische Ansicht einer weiteren bevorzugten Ausführungsform einer Atemmaske mit einem, mit einer Lokalfaltenbalgstruktur versehenen Masken-Dichtkissen;
**Fig. 12** eine perspektivische Ansicht der Atemmaske gem. Fig. 11 von unten;
**Fig. 13** eine vereinfachte Schnittansicht durch die auf der Oberlippe aufsitzende Dichteinrichtung, zur Erläuterung des weitgehend glattwandigen Übergangs der Dichtlippe in den Hartschalen-Maskenkörper,
**Fig. 14a** eine vereinfachte Schnittansicht durch eine Dichtlippe mit integrierter Leckage-Öffnung;
**Fig. 14b** eine vereinfachte Schnittansicht durch eine Dichtlippe mit integrierter Leckage-Öffnung, jedoch mit partiell vom Maskenrahmen begrenzten Strömungsweg;
**Fig. 14c** eine vereinfachte Schnittansicht durch eine Dichtlippe mit integrierter Leckage-Öffnung, mit in den Maskenrahmen eingeformten und in den Dichtlippenrahmenabschnitt einfließendem Strömungsweg;
**Fig. 14d** eine vereinfachte Schnittansicht durch eine Dichtlippe mit integrierter Leckage-Öffnung, jedoch mit miteinander fluchtenden Kanälen in der Hartschale sowie der Dichtlippeneinrichtung;
**Fig. 14e** eine vereinfachte Schnittansicht durch eine Dichtlippe mit integrierter Leckage-Öffnung, jedoch mit von innen an eine Durchgangsöffnung latzartig hochgeführtem Abschnitt und darin ausgebildeter Durchgangsöffnung;
**Fig. 15** vereinfachte Skizzen bevorzugter Querschnitte der Strömungswege;
**Fig. 16** eine vereinfachte Prinzipskizze zur Erläuterung bevorzugter Leckagezonen.

Die in Fig.1 dargestellte, als Maskenkissen 1 ausgebildete Dichtlippeneinrichtung ist aus einem elastomeren Material, hier transparenter Silikonkautschuk gefertigt.

Das Maskenkissen 1 umfaßt eine um eine Nasenaufnahmeöffnung 2 umlaufende, Dichtlippe 3. Die Dichtlippe 3 weist eine bei der hier dargestellten Ausführungsform konvex gekrümmte Außenfläche auf.

Die Dichtlippe 3 ist derart ausgebildet und angeordnet, daß diese aus sich selbst heraus Zonen unterschiedlicher Tragfähigkeit aufweist. Bei der hier dargestellten Ausführungsform wird dies durch eine entgegen der Applikationsrichtung Z nachgiebige Aufhängung der zur Auflage auf dem Nasenrücken vorgesehenen Dichtlippenzone a (siehe Skizze K1) erreicht.

Zusätzlich hierzu ist die Dichtlippe 3 im Bereich der den Nasenflügeln benachbarten Zone b1, b2 (Skizze K1) derart ausgebildet, daß diese hier eine höhere Tragfähigkeit aufweist. Hierdurch wird eine Schwenkbarkeit des Maskenkissens um eine Adaptionsachse X erreicht die quer durch das Maskenkissen in dem in der Skizze K1 durch den Buchstaben e gekennzeichneten Bereich verläuft.

Diese höhere Tragfähigkeit wird hier durch Pad-artig verdickte Zonen 4 erreicht die hier in vorteilhafter Weise mondsichelartig in die Dichtlippe 3 auslaufen. Die Zonen 4 höherer Tragfähigkeit sind jeweils an einem ebenfalls vergleichsweise dickwandigen Stützwandungsabschnitt 5 abgestützt. Die Stützwandungsabschnitte 5 bilden ebenfalls einen integralen Bestandteil des Maskenkissens 1 und sind als dickwandige Zonen der vorderen sich in den Zonen b1,c und b2 erstreckenden Umfangswandung verwirklicht.

Die Nachgiebigkeit entgegen der Applikationsrichtung nimmt entlang der Dichtlippe 3 ausgehend von den Zonen 4 hoher Tragfähigkeit bis zum nasenrückenseitigen Zenit Q ab und steigt dann langsam bis zum Außenrandpunkt R an.

Die entgegen der Applikationsrichtung Z nachgiebige Lagerung der Zone a der Dichtlippe 3 wird bei der dargestellten Ausführungsform durch eine Faltenbalgstruktur mit unterschiedlicher Tragfähigkeit erreicht.

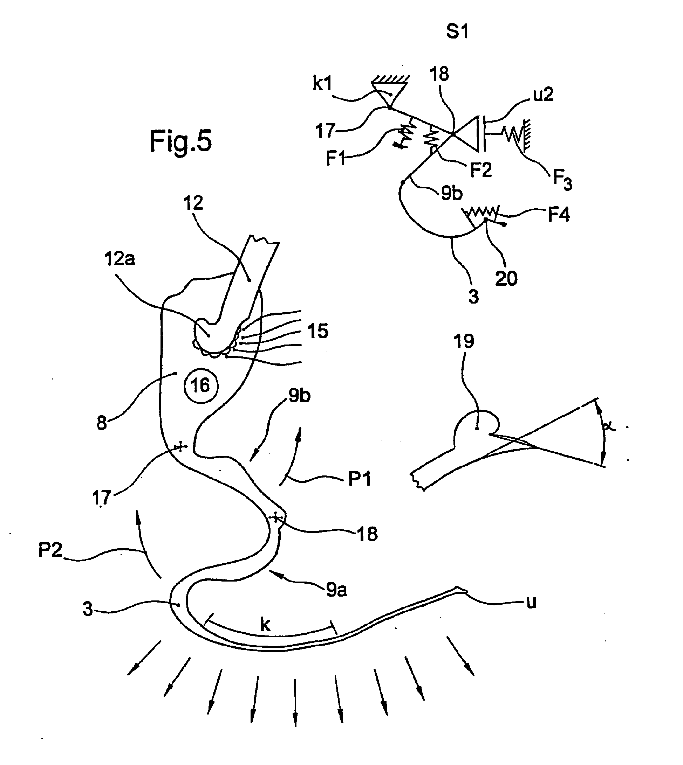

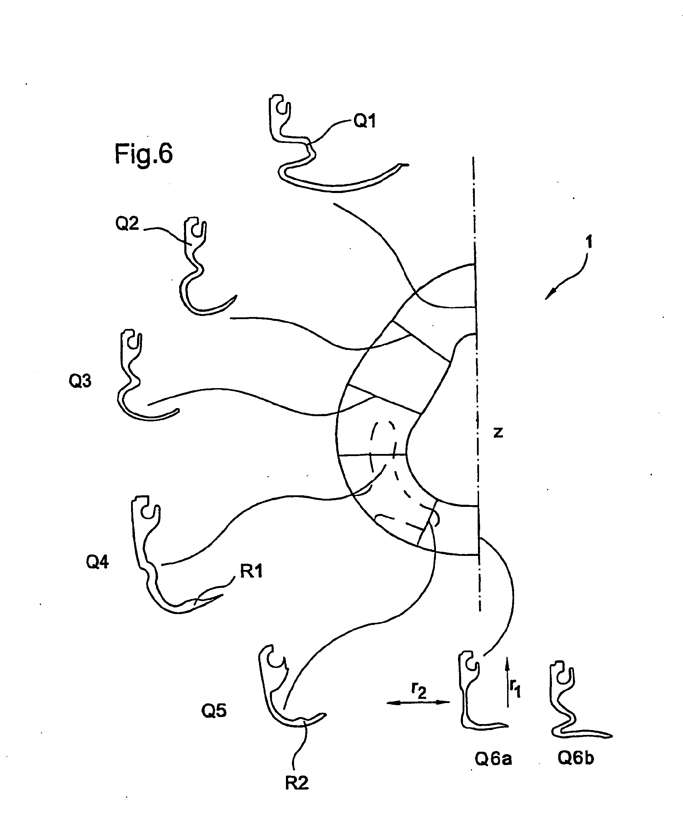

Die unterschiedliche Tragfähigkeit wird hier sowohl durch die Geometrie und Anordnung der Faltenbalgstruktur als auch durch einen besonderen Wandstärkenverlauf erreicht. Auf diesen Wandstärkenverlauf wird insbesondere in Verbindung mit den Figuren 5 und 6 noch näher eingegangen werden.

Das Maskenkissen 1 umfaßt weiterhin einen umlaufenden Rahmen 8 welcher mit einer Befestigungsprofilierung versehen ist, die komplementär zu einem, an einem Maskenbasiskörper (nicht dargestellt) ausgebildeten Befestigungsprofilabschnitt ausgebildet ist.

Die Umfangslänge des Rahmens 8 sowie deren Verlauf um eine zentrale Achse z des Maskenkissens 1 sind derart gewählt, daß in Verbindung mit einem Maskenbasiskörper eine definierte Vorspannung des Maskenkissens 1 insbesondere tendenzielle Hervorwölbung nach außen erreicht wird.

Die Wanddicke der Dichtlippe 3 bewegt sich bei der dargestellten Ausführungsform im Bereich von 0,6 bis 3,2 mm.

Der Verlauf der die Nasenaufnahmeöffnung 2 umsäumenden Umfangskante u ist derart gewählt, daß zwei leicht zur Maskenachse z einwärts vorkragende Segmente s1, s2 (Skizze K3) gebildet werden.

Durch die Abstimmung des Verlaufes der Umfangskante u auf die konvexe Wölbung der Dichtlippe 3 kann ein Verformungsverhalten erreicht werden, bei welchem eine Aufweitung der Dichtlippe im Bereich der Umfangskante zu einer definiert erhöhten Flächenpressung auf dem Gesicht des Maskenanwenders führt.

In einem vorderen stirnseitigen Mittenbereich c ist eine weitere Zone mit verminderter Tragfähigkeit ausgebildet. Diese definiert verminderte Tragfähigkeit wird hier durch eine deutlich verminderte Wanddicke herbeigeführt. Es ist auch möglich in der Zone c lokale Falten- oder Rollbalgstrukturen vorzusehen.

Eine besonders bevorzugte Ausführungsform einer Dichtlippeneinrichtung ist dadurch gegeben, daß in diese Auslaßöffnungen 50 integriert sind über welche ein definierter Gasstrom aus dem Inneren der Atemmaske abströmen kann. Diese Auslaßöffnungen weisen vorzugsweise wie in der Skizze K2 dargestellt einen sich nach außen konisch verjüngenden Querschnitt auf.

Vorzugsweise sind diese Auslaßöffnungen zunächst beispielsweise durch einen dünnen Film verschlossen und werden bedarfsgerecht beispielsweise durch Nadelpunktion geöffnet. Wie aus dieser Skizze weiter ersichtlich ist das Maskenkissen 1 über einen Rahmenabschnitt 8 an einem Maskenbasiskörper 12 anbringbar. Hierzu ist vorzugsweise eine Umfangswulststruktur mit häkelnadelartigem Querschnitt und gerundeten Kanten, vorgesehen.

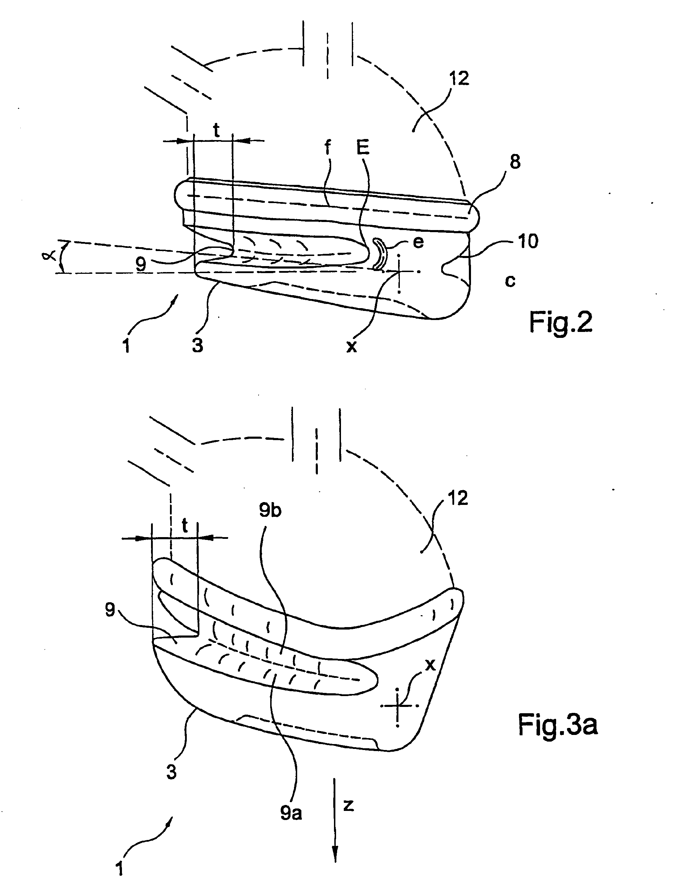

In Fig. 2 ist eine Seitenansicht einer weiteren Ausführungsform eines Maskenkissens 1 dargestellt. Bei dieser Ausführungsform erstreckt sich der Rahmen 8 im wesentlichen in einer ebenen Rahmenaufspannfläche f.

Das Maskenkissen 1 weist ebenfalls im Nasenrückendichtbereich eine lokale Faltenbalgstruktur 9 auf durch welche eine nachgiebige Aufhängung der Dichtlippe 3 erreicht wird.

Im vorderen Bereich c (Definition analog zur Skizze K1 in Fig.1) ist ebenfalls ein Falteneinzug 10 vorgesehen. Durch die derart getroffene Anordnung wird eine Adaptions- und Artikulationsachse X bzw. ein Momentandrehpol definiert um welche bzw. welchen die Dichtlippe 3 elastisch verkippt werden kann. Die Anordnung ist hier derart getroffen, daß Verkippungswinkel α im Bereich von bis zu 15° möglich sind. Die Dichtlippe 3 kann sich neben der Kippbewegung selbst ebenfalls entsprechend der Gesichtstektur individuell verformen. Insbesondere wird die Umfangskante u der Nasenaufnahmeöffnung gedehnt.

Bei größeren Verkippungswinkeln wird hier die Faltenbalgstruktur als Anschlageinrichtung wirksam und begrenzt in ebenfalls elastisch nachgiebiger Weise eine weiteres Eintauchen des Nasenrückens in das Maskenkissen 1.

Die Faltenbalgstruktur 9 weist im Bereich des nasenrückenseitigen Endes die größte Einzugstiefe t auf. Diese Einzugstiefe nimmt bis zum vorderen Ende E der Faltenbalgstruktur 9 allmählich ab.

Bei der hier dargestellten Ausführungsform ist der Auslauf der Faltenbalgstuktur 9 gerundet ausgebildet. In vorteilhafter Weise ist im Bereich des vorderen Endes E des Falteneinzuges eine Mikrofaltenstruktur e ausgebildet die einen gleichmäßigeren Abbau von Materialspannungen in diesem Bereich bewirkt. Hierdurch wird eine verbesserte Haltbarkeit erreicht.

In Fig. 3a ist eine weitere Ausführungsform eines Maskenkissens 1 in Verbindung mit einem lediglich angedeuteten Maskenkörper 12, dargestellt.

Auch bei dieser Ausführungsform ist eine lokale Faltenbalgstruktur 9 vorgesehen. Die Geometrie dieser Faltenbalgstruktur 9 ist derart gewählt, daß die Faltenflanken 9a, 9b zueinander geneigt verlaufen. Insgesamt ist die Einzugstiefe t auch hier im Bereich des nasenrückenseitgen Endes größer als in den übrigen Bereichen. Das Maskenkissen 1 definiert ebenfalls eine Adaptionsachse X die im Bereich der Zonen c1, b2 bzw. c auf Höhe der Nasenflügel eines Maskenanwenders, verläuft.

Das Maskenkissen 1 weist aufgrund der hier vorgesehenen Aufhängung der Dichtlippe 3 an einer lokalen Faltenbalgstruktur 9 ebenfalls im Bereich der den Nasenrücken abdichtenden Zone a eine höhere Nachgiebigkeit in entgegen der Applikationsrichtung Z auf.

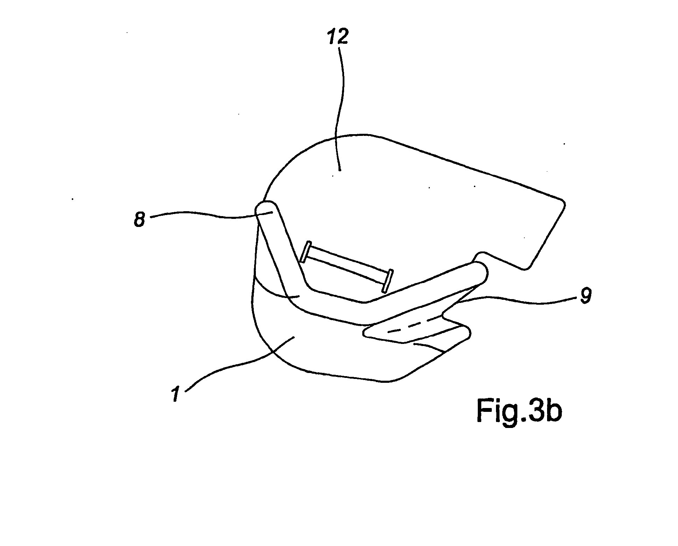

In Figur 3b ist eine weitere Ansicht einer Atemmaske mit einem erfindungsgemäßen Maskenkissen 1 dargestellt. Das Maskenkissen 1 ist hier über einen Rahmen 8 an einem Maskenbasiskörper 12 befestigt. Im Bereich des den Nasenrücken abdichtenden Abschnitts der Dichtlippe 3 ist hier eine Faltenbalgstruktur 9 vorgesehen. Abweichend von den vorangehend beschriebenen Ausführungsformen ist hier auch die Umfangswandung des Maskenkissen auch im Bereich des Faltenbalgstruktur 9 dünnwandig ausgebildet. Das Maskenkissen 1 ist unter erheblicher Weitung und Dehnung des Rahmens 8 auf den Maskenbasiskörper 12 aufgespannt.

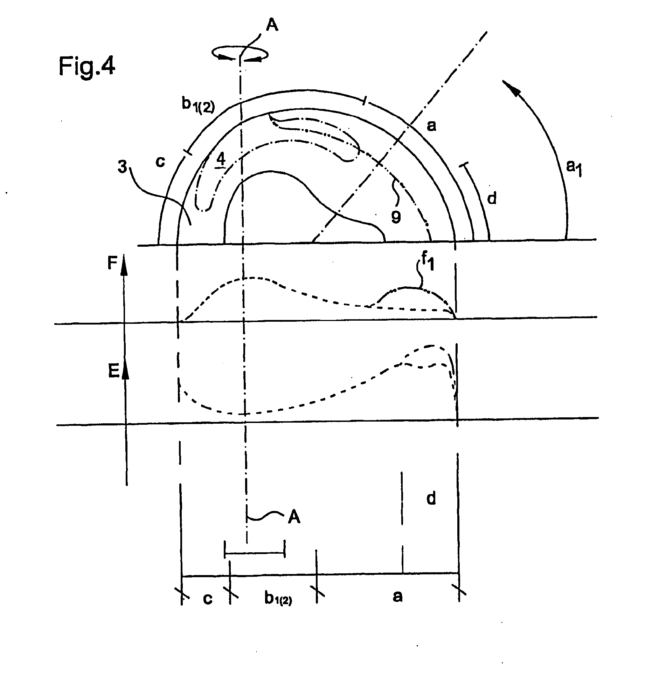

In Fig. 4 ist in Verbindung mit einer Draufsicht auf eine Hälfte der Dichtlippe 3 Die Tragfähigkeit sowie die Nachgiebigkeit des Maskenkissens1 veranschaulicht.

Die geringste Nachgiebigkeit E des Maskenkissens 1 herrscht im Bereich b. Die höchste Nachgiebigkeit herrscht im Bereich a welcher den Nasenrücken und die oberen Seitenflanken der Nase des Maskenanwenders abdeckt. Im Bereich c herrscht zusätzlich zur Nachgiebigkeit entgegen der Applikationsrichtung Z auch eine größere Nachgiebigkeit in radialer Richtung.

Die Adaptionsachse A verläuft durch die Zone b höherer Tragfähigkeit. Bei Überschreiten einer vorbestimmten Eindringtiefe in das Maskenkissen1 wird die Faltenbalgstruktur in einem Bereich d als Anschlageinrichtung wirksam und verursacht hierbei einen raschen Anstieg der über die Dichtlippe 3 übertragenen Druckkraft F wie dies durch den Strichpunktlinienabschnitt f1 angedeutet ist.

Die besonderen mechanischen Eigenschaften der Aufhängung der Dichtlippe 3 werden vorzugsweise durch die Wanddicke im Bereich der Faltenbalgstruktur 9 sowie durch die Einzugstiefe und Ausrichtung der Faltenbalgflanken 9a 9b (Fig. 3a) bestimmt.

In Fig. 5 ist eine bevorzugte Gestaltung der Wanddicken der Balgstruktur 9 dargestellt. Die Befestigung des Maskenkissens 1 an einem Maskenbasiskörper 12 erfolgt hier über eine gerundete Profilstruktur 12a die sich entlang des Rahmens 8 erstreckt. Bei der dargestellten Ausführungsform weist diese Profilstruktur 12a einen häkelnadelartigen Querschnitt auf. Zumindest abschnittsweise sind im Bereich der Berührungszone zwischen Rahmen 8 und Maskenbasiskörper 12 umlaufende Profilstege 15 vorgesehen, durch welche selbst im Falle einer erheblichen Relativbewegung eine sichere Abdichtung erreicht wird.

Unterhalb des Rahmens 8 befindet sich zunächst ein dickwandiger Abschnitt 16 welcher sich allmählich zu einer ersten Balggefenkstelle 17 hin verjüngt. An diese Balggelenkstelle 17 schließt sich ein erster Balgflankenschenkel 9b an. Dieser Balgflankenschenkel 9b weist im Querschnitt Zonen unterschiedlicher Wanddicke auf und erstreckt sich bis zu einer Balginnengelenkstelle 18 die durch eine dünnwandige Zone definiert ist.

An die Balginnengelenkstelle 18 wiederum schließt sich ein zweiter Balgflankenschenkel 9a an welcher ebenfalls Zonen unterschiedlicher Wanddicke aufweist.

An dem zweiten Balgflankenschenkel 9a ist letztendlich die Dichtlippe 3 aufgehängt. Die Dichtlippe 3 ist hier im Vergleich zur Balgstruktur 9 extrem dünnwandig ausgebildet.

Der hier dargestellte Querschnitt des Dichtkissens entspricht qualitativ dem Dichtkissenquerschnitt im Bereich der in Fig. 4 als a1 gekennzeichneten Zone.

Im Rahmen der Applikation des Maskenkissens auf das Gesicht eines Maskenanwenders sitzt zunächst die Dichtlippe 3 auf dem Gesicht auf. Anschließend federn die Balgflankenschenkel 9a und 9b entsprechend der Eintauchtiefe des Nasenrückens wie durch die Pfeile P1 und P2 dargestellt, ein. Im Falle eines besonders tiefen Eindringens des Nasenrückens gelangt ggf. die Innenfläche der Dichtlippe 3 im Bereich der Zone k mit der ihr zugewandten Innenfläche des Balgflankenschenkels 9b in Berührungskontakt. Der Balgflankenschenkel 9b wiederum kann auf der ihm zugewandten Außenfläche des Balgflankenschenkels 9a aufsitzen.

Die Kinematik der Dichtkissenaufhängung wird anhand der beigefügten Funktionsskizze S1 deutlich. So kann der Rahmen als feste Einspannung K1 betrachtet werden, an welcher der Balgflankenschenkel 9b an der Gelenkstelle 17 schwenkbar gelagert ist. Die Eigenelastizität des elastomeren Materials im Bereich der Gelenkstelle 17 ist durch die Feder F1 symbolhaft angedeutet.

Die Balginnengelenkstelle 18 hat ebenfalls ein eigenelastisches Verhalten das durch die Feder F2 angedeutet ist. Das Loslager K2 sowie die Feder F3 sind dadurch bedingt, daß es sich hier um eine räumliche, ringartige Struktur handelt die auch in radialer Richtung Kräfte aufnimmt.

An die Gelenkstelle 18 schließt sich der Balgflankenschenkel 9b und an diesen die membranartige Dichtlippe 3, an.

Entlang der inneren Umfangskante u ist eine Mikrodichtlippenstruktur ausgebildet durch welche eine dünn auslaufende Dichtkante geringfügig nach außen vorgespannt ist. Diese Mikrodichtlippenstruktur weist einen Wulstabschnitt 19 auf durch welchen die Einreißfestigkeit der Dichtlippe 3 erhöht wird.

Die Mechanik dieser Mikrodichtlippenstruktur ist in der Skizze S1 durch eine Feder F4 und eine Gelenkstelle 20 angedeutet. Die derart elastisch aufgehängte Dichtlippe kann wie durch die kleinen Pfeile angedeutet infolge des im Inneren der Maske herrschenden Maskeninnendrucks flexibel gegen die Gesichtsfläche des Maskenanwenders gedrängt werden.

Wie aus Fig. 6 ersichtlich, weist das Maskenkissen 1 vorzugsweise entlang seines Verlaufes um die Maskenachse Z unterschiedliche Querschnitte auf wie dies hier skizzenhaft angedeutet ist.

Der Querschnitt Q1 weist eine deutliche Gelenkcharakteristik mit Anschlageigenschaften auf.

Der Querschnitt Q2 weist bereits eine geringere Gelenkcharakteristik und einen geringeren Falteneinzug auf.

Im Bereich der Querschnitte Q3, Q4 tritt die Faltenbalgeigenschaft noch weiter zurück.

Die höhere Tragfähigkeit der Querschnitte Q4 und Q5 wird durch lokale linsenartig bis in die Dichtlippe hinein auslaufende Verdickungen R1, R2 erreicht. In den Zonen hoher Tragfähigkeit kann ggf. wie hier geschehen, auf die Faltenbalgstruktur verzichtet werden.

Die Querschnitte Q6a oder Q6b sind derart gestaltet, daß eine Nachgiebigkeit in die hier angedeuteten Richtungen r1 und r2 besteht. Hierdurch wird unmittelbar neben den tragenden Zonen eine verbesserte Adaptionsfähigkeit hinsichtlich der Oberlippentektur erreicht.

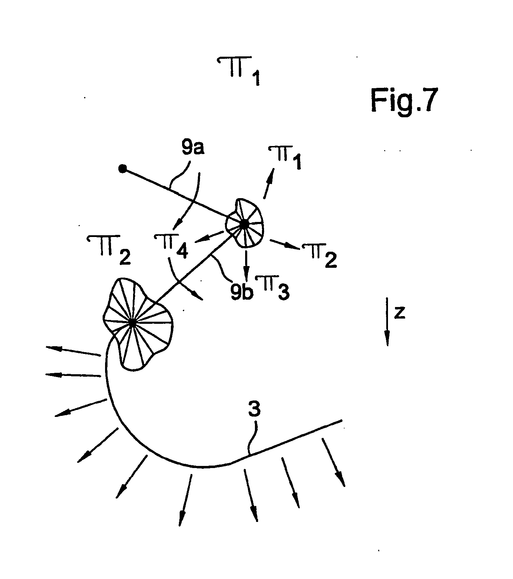

Es ist auch möglich, die Faltenbalgstuktur 9 dünnwandig auszubilden. Die Kinematik einer derartigen Struktur ist in Fig.7 skizzenhaft dargestellt. Die membranartige Dichtlippe 3 ist hier an zwei Schenkeln (Balgflankenschenkel 9a, 9b) aufgehängt. Bei dieser Ausführungsform ist auch bei geringen Maskeninnendrücken eine hohe Adaptionsfähigkeit gewährleistet. Durch die hier skizzenhaft eingezeichneten Polardiagramme Π1 Π2 ist das Elastizitätsverhalten unter Bezugnahme auf eine Einheitskraft für alle Belastungswinkel dargestellt. Wie erkennbar ist durch die erfindungsgemäße Aufhängung der Dichtlippe 3 nicht nur entgegen der Applikationsrichtung Z sondern auch in sämtliche anderen Richtungen eine definierte Adaptionsfähigkeit gegeben. Die Ortsvektoren π1, π2, π3 und π4 verdeutlichen diese Nachgiebigkeit im Bereich der Balginnengelenkstelle. Die Bewegungsmöglichkeiten der Balginnengelenkstelle 18 übertragen sich (unter Einfluß der Maskenkissen-Umfangskräfte) auch auf den Aufhängungsbereich der Dichtlippe 3.

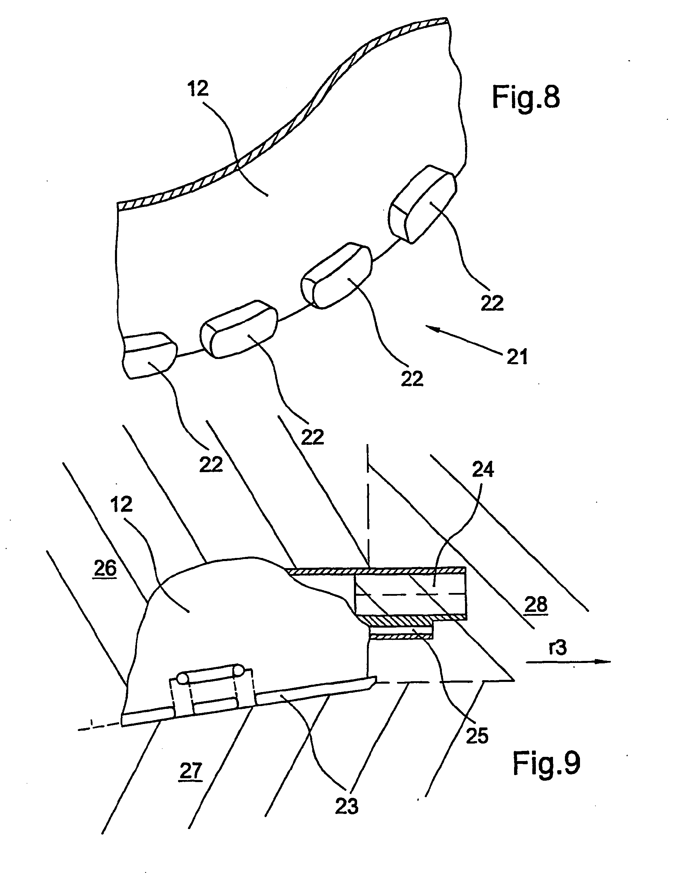

In Figur 8 ist vereinfacht eine seitens eines Maskenbasiskörpers 12 vorgesehene Profilstruktur 21 dargestellt, durch welche auf vorteilhafte Weise eine zuverlässige Fixierung des Maskenkissens in Umfangsrichtung erreicht wird. Bei der dargestellten Ausführungsform sind hierzu eine Vielzahl einzelner Fixiervorsprünge 22 entlang des Umfangs des Maskenbasiskörpers 12 vorgesehen. Alternativ hierzu oder auch in Kombination mit dieser Maßnahme ist es auch möglich, weitere Fixiereinrichtungen insbesondere zapfenartige Vorsprünge, vorzusehen.

In Fig. 9 ist stark vereinfacht der Aufbau eines Formwerkzeuges zur Herstellung des Maskenbasiskörpers 12 dargestellt. Aufgrund der Durchbrechung des Umfangswulstes 23 im Bereich der jeweiligen Gurtschlaufen wird es möglich die Gurtschlaufen integral mit dem Maskenbasiskörper 12 zu Spritzen ohne daß hierbei Bedarf nach Formschiebern besteht.

Bei der hier skizzierten Ausführungsform des Maskenbasiskörpers 12 ist parallel zu einem Atemgaskanal 24 ein Nebenkanal 25 vorgesehen über welchen beispielsweise eine Druckmessung erfolgen kann ohne daß hierbei Querschnittsverengungen auftreten.

Das Werkzeug ist hier dreiteilig aufgebaut und umfaßt eine obere Werkzeughälfte 26, eine untere Kemwerkzeughälfte 27 und einen Formschieber 28 welcher in Richtung r3 von dem Atemgaskanal 24 abziehbar ist.

Obgleich die Erfindung vorangehend unter Bezugnahme auf bevorzugte Ausführungsbeispiele beschrieben wurde bei welchen ein einziger Falteneinzug vorgesehen ist welcher sich nicht um den gesamten Umfang des Maskenkissens erstreckt, ist die Erfindung nicht auf derartige Ausführungsbeispiele beschränkt.

Beispielsweise ist es möglich die Faltenbalgstruktur mit mehreren Falteneinzügen auszustatten von welchen sich ggf. einer oder mehrere um den gesamten Umfang des Maskenkissens erstrecken.

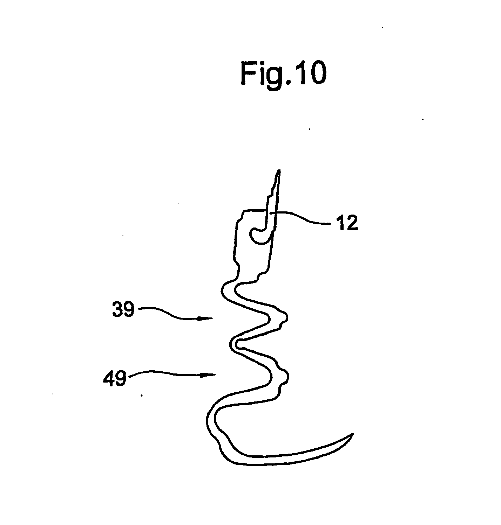

Ein Beispiel für eine entsprechende Querschnittsgestaltung ist in Fig.10 dargestellt. Das hier an einem nur abschnittsweise angedeuteten Maskenbasiskörper 12 über eine im Querschnitt häkelnadelartige Umfangswulststruktür befestigte Maskenkissen 1 weist zwei lokale Falteneinzüge 39, 49 auf. Die Wandung dieser lokalen Falteneinzüge 39, 49 ist im Hinblick auf eine definierte Gelenk- und Nachgiebigkeitscharakteristik abgestimmt.

Die Dichtlippe 3 ist bei dieser Ausführungsform vergleichsweise dickwandig ausgebildet. Dieser Querschnitt eignet sich insbesondere für Silikonkautschukmaterial mit äußerst geringer Shorehärte.

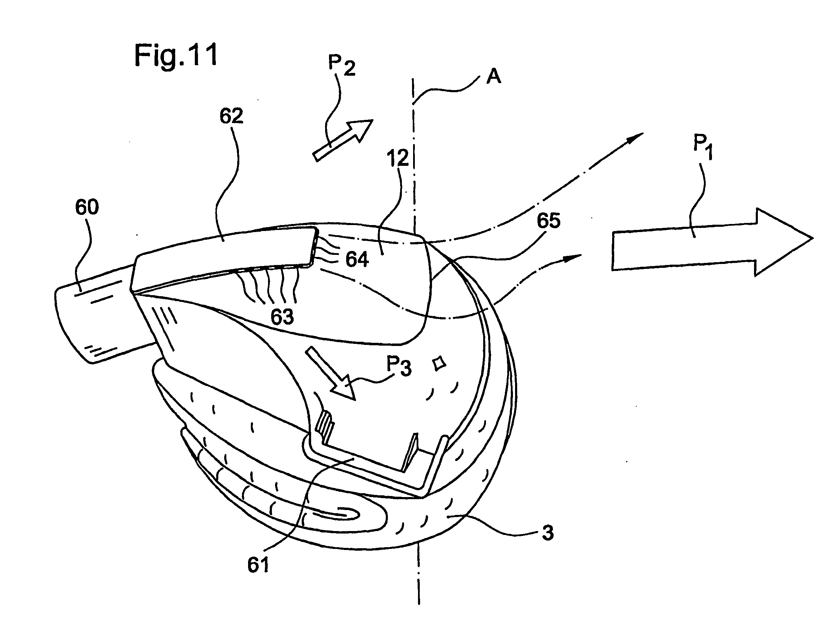

Die in Fig. 11 dargestellte Atemmaske umfaßt einen aus einem vorzugsweise volltransparenten, thermoplastischen Kunststoffmaterial gefertigten Maskenbasiskörper 12. In einem in Applikationsposition der Maske dem Stirnbereich des Maskenanwenders benachbarten Wandungsabschnitt ist ein Anschlußstutzen 60 vorgesehen, welcher hier einen Polygon-Querschnitt aufweist.

Über eine, hier nicht sichtbare, Umfangswulststruktur ist an dem Maskenbasiskörper 12 die Dichtkisseneinrichtung 3 befestigt. Die Dichtkisseneinrichtung 3 weist eine lokal vom oberen Endbereich bis zu einer Adaptionsachse A verlaufende Faltenbalgstruktur auf. Im Bereich der Adaptionsachse A sind zu beiden Seiten des Dichtkissens Zonen höherer Tragfähigkeit ausgebildet, die durch dickwandigere und sphärisch gewölbte Zonen der Dichtkisseneinrichtung gebildet sind.

Zur Anbringung der Atemmaske auf dem Gesicht eines Maskenanwenders sind zu beiden Seiten der Maske Befestigungseinrichtungen 61 vorgesehen, über welche ein Kopfband mit der Atemmaske gekoppelt werden kann.

Der Maskenkörper 12 ist auf seiner Oberseite mit einem Vorsprung 62 versehen, durch welchen der Maskenkörper insgesamt ausgesteift wird, wodurch sich ein verbessertes Körperschallverhalten ergibt.

Ebenfalls im Bereich der Oberseite des Maskenkörpers 12 sind eine Vielzahl Auslaßöffnungen 63, 64 vorgesehen, über welche eine geräuscharme, gerichtete Abströmung von teilweise verbrauchter Atemluft aus dem Inneren der Maske erfolgen kann. Die Abströmung dieses Leckagegasstromes wird durch eine spoilerartig ausgebildete Abrißkante 65 unterstützt. Die Öffnungen 64 strahlen im wesentlichen entlang der durch den Pfeil P1 gekennzeichneten Richtung. Die Öffnungen 63, die auch auf der hier nicht sichtbaren, gegenüberliegenden Seite des Vorsprunges 62 vorgesehen sind, strahlen in die Richtungen P2 und P3.

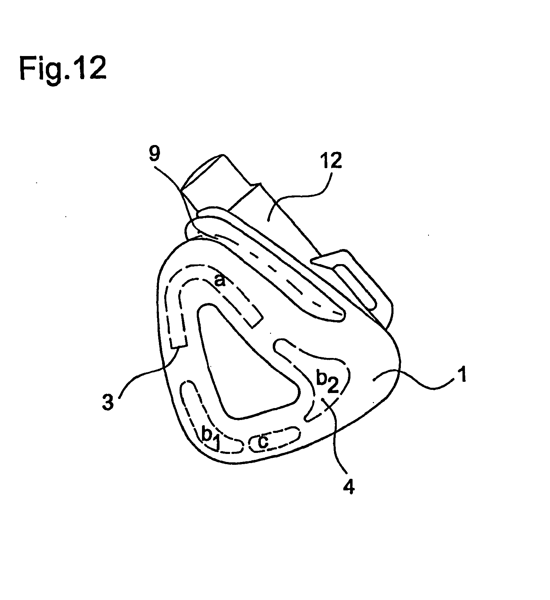

In Fig. 12 ist die Atemmaske gem. Fig. 11 aus einer schräg von unten auf die Zone 4 hoher Tragfähigkeit gerichteten Blickrichtung dargestellt. Erkennbar ist hier neben der lokalen Faltenbalgstruktur 9 auch der auf dem Gesicht des Maskenanwenders aufsitzende Bereich der Dichtlippe 3. Im Bereich der Zone a zeichnet sich die Maske durch eine hohe Adaptionsfähigkeit an unterschiedliche Nasenrückenhöhen aus. In den Zonen b1 und b2 stützt sich das Maskenkissen 1 definiert auf dem Gesicht des Maskenanwenders ab. Im Bereich c wiederum herrscht eine höhere Nachgiebigkeit und höhere Adaptionsfähigkeit an unterschiedliche Oberlippentekturen.

Ist das Maskenkissen derart gestaltet, daß sich infolge des im Rahmen einer Überdruckbeatmung ergebenden Maskeninnendrucks eine Entlastung im Bereich der Zonen b1 und b2 ergibt. Die Flächenpressung des Maskenkissens im Bereich der Zonen a und c wird im wesentlichen durch den Maskeninnendruck bestimmt. In Umfangsrichtung weist das Dichtkissen 1 eine hohe radiale Steifigkeit auf, wodurch die Oszillationsneigung des Dichtkissens bei alternierenden Beatmungsdrücken deutlich reduziert ist.

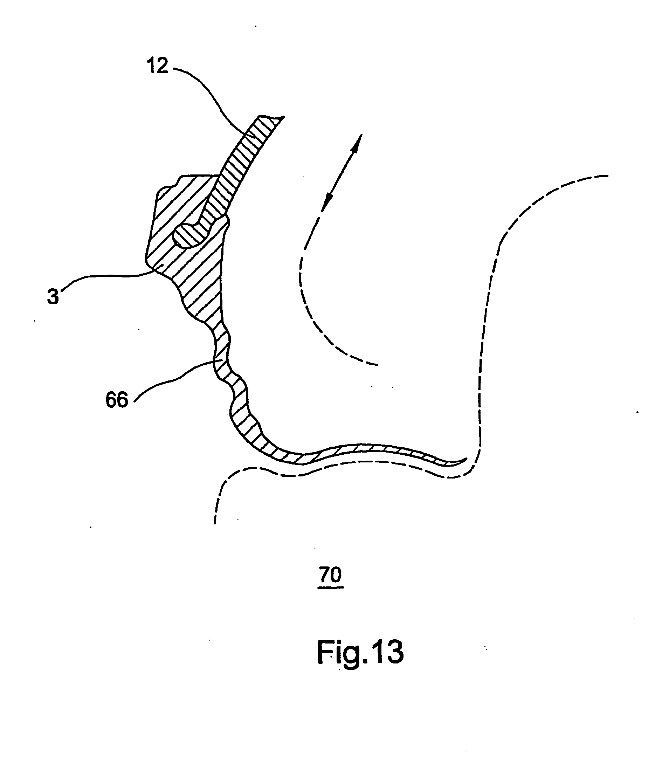

In Fig. 13 ist stark vereinfacht eine Schnittansicht durch den auf der Oberlippe 70 eines Maskenanwenders aufsitzenden Bereich der Dichtlippeneinrichtung 3 dargestellt. In einem Übergangsbereich von der Dichtkisseneinrichtung in den Hartschalenkörper 12 ist eine derartige Gestaltung der Querschnitte von Dichtkisseneinrichtung 3 und Hartschalenkörper 12 getroffen, daß sich ein im wesentlichen glatter Übergang der jeweiligen Innenflächen ergibt. Hierdurch wird unmittelbar im Bereich der Nasenöffnungen des Maskenanwenders ein günstiger Strömungsweg gewährleistet.

Wie angedeutet dargestellt, ist auch hier eine lokale Faltenbalgstruktur 66 vorgesehen, durch welche eine verbesserte Adaptionsfähigkeit an unterschiedliche Oberlippentekturen gewährleistet ist.

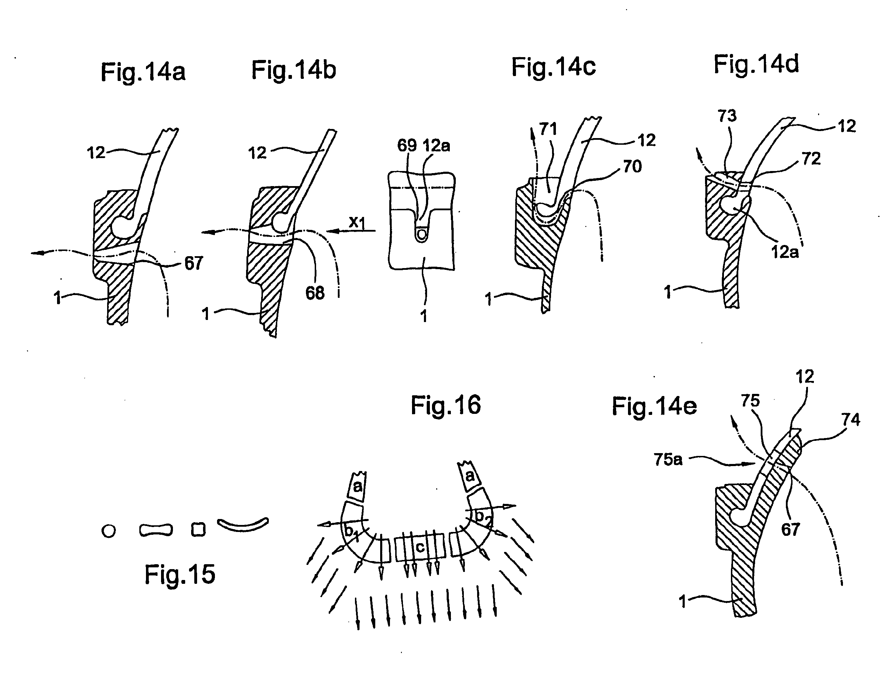

In Fig. 14a ist abschnittsweise der Übergangsbereich zwischen dem Hartschalenkörper 12 und der Dichtkisseneinrichtung 1 dargestellt. Unmittelbar in die Dichtkisseneinrichtung 1 ist eine Leckageöffnung 67 eingeformt, die hier einen sich in Austrittsrichtung verjüngenden Querschnitt aufweist. Die Querschnitte dieser Leckageöffnung 67 weisen vorzugsweise die in Fig. 15 skizzierte Gestalt auf.

In Fig. 14b ist eine weitere Ausführungsform einer in die Dichtkisseneinrichtung 1 integrierten Leckageöffnung 68 dargestellt. Bei der hier gezeigten Ausführungsform erstreckt sich eine durch den Hartschalenkörper 12 gebildete Wandung in den Strömungsweg hinein. Diese Ausführungsform läßt sich in besonders vorteilhafter Weise reinigen, da nach Abnahme der Dichtkisseneinrichtung 1 von dem Hartschalenkörper 12 der Strömungsweg großflächig freiliegt. In der unmittelbar nebenstehend gezeigten Skizze ist eine Ansicht dieses Dichtkissendetails aus der als x1 gekennzeichneten Blickrichtung gezeigt. Wie erkennbar, erstreckt sich der unter der Umfangswulst 12a des Hartschalenkörpers 12 teilweise in die in der Dichtkisseneinrichtung 1 gebildete Ausnehmung 69 hinein.

Gem. der in Fig. 14c dargestellten Ausführungsform ist im Bereich einer Trennfuge zwischen Hartschalenkörper 12 und Dichtkisseneinrichtung 1 in dem Hartschalenkörper ein Rinnenabschnitt 70 ausgebildet, über welchen eine Gasabströmung, wie durch Strichpunktlinien angedeutet, erfolgen kann. Der Austrittsbereich des Rinnenabschnitts 70 mündet, wie hier dargestellt, in einen durch die Dichtkisseneinrichtung 3 und den Hartschalenkörper 12 gemeinsam definierten Auslaßkanal 71.

Bei der gem. Fig. 14d dargestellten Ausführungsform ist in dem Hartschalenkörper 12 wenigstens ein Auslaßkanal 72 vorgesehen, der in einen fluchtenden, in der Dichtungseinrichtung 1 ausgebildeten Ablaßkanal 73 übergeht.

In Fig. 14e ist eine Ausführungsform einer Leckageeinrichtung dargestellt, bei welcher ein mit der Dichtkisseneinrichtung 1 integraler Wandungsabschnitt 74 von innen an einen Auslaßöffnungsbereich 75 des Hartschalenkörpers 12 herangeführt ist. Dieser Wandungsabschnitt 74 ist hier mit einer Auslaßöffnung 67 versehen, die sich in Austrittsrichtung konisch verjüngt und koaxial zu einer vorzugsweise erheblich größeren Auslaßöffnung 75a angeordnet ist.

Die in Verbindung mit den Fig. 14a bis 14e beschriebenen Strömungswege weisen vorzugsweise wenigstens einen der in Fig. 15 skizzierten Querschnitte auf.

In Fig. 16 ist ein bevorzugter Anbringungsort für die gemeinsam mit der Dichtkisseneinrichtung 1 oder auch separat hiervon vorgesehenen Abströmöffnungen angedeutet. Vorzugsweise erfolgt die Abströmung im Bereich der Zone c in Kombination mit den Zonen b1 und b2, wobei jedoch im Bereich c vorzugsweise größere Volumenströme zugelassen sind.

## Patentansprüche

1. Dichtlippeneinrichtung für eine Atemmaske mit
einer Aufnahmeöffnung zur Aufnahme wenigstens des Nasenspitzenbereiches eines Maskenanwenders,
einer aus einem elastomeren Material gebildeten, um die Aufnahmeöffnung umlaufenden und den Nasenrücken überquerenden Dichtlippe (3),
die eine zur Auflage auf dem Gesicht eines Maskenanwenders vorgesehenen Auflagezone aufweist, wobei
die Dichtlippe (3) derart elastisch nachgiebig angeordnet ist, dass sich für die den Bereich des Nasenrückens abdichtende Dichtlippenzone (a) eine höhere Nachgiebigkeit ergibt als für die Dichtlippenzone (b1, b2; c),
welche in Applikationsposition der Atemmaske den Nasenflügeln und/oder der Oberlippe eines Maskenanwenders benachbart ist,
wobei die Dichtlippe (3) im Bereich einer zur Abdichtung des Nasenrückenbereichs vorgesehenen Zone (a) an einer Faltenbalgstruktur (9) aufgehängt ist, **dadurch gekennzeichnet, dass** die Faltenbalgstruktur (9)
eine durch unterschiedliche Wandstärken definierte Gelenkcharakteristik aufweist.

2. Dichtlippeneinrichtung nach Anspruch 1, **dadurch gekennzeichnet, dass** die Faltenbalgstruktur (9) eine Anschlageinrichtung bildet.

3. Dichtlippeneinrichtung nach wenigstens einem der Ansprüche 1 bis 2, **dadurch gekennzeichnet, dass** die Faltenbalgstruktur (9) mehrere Falteneinzüge aufweist.

4. Dichtlippeneinrichtung nach wenigstens einem der Ansprüche 1 bis 3, **dadurch gekennzeichnet, dass** wenigstens ein Falteneinzug sich vom Nasenrückenbereich bis in einen, in Gebrauchsposition der Maske den Nasenflügeln benachbarten Bereich hinein erstreckt.

5. Dichtlippeneinrichtung nach wenigstens einem der Ansprüche 1 bis 4, **dadurch gekennzeichnet, dass** wenigstens ein Falteneinzug sich um den gesamten Umfang der Dichtlippeneinrichtung herum erstreckt.

6. Dichtlippeneinrichtung nach wenigstens einem der Ansprüche 1 bis 5, **dadurch gekennzeichnet, dass** die auf die Applikationsrichtung (Z) bezogene Nachgiebigkeit der Dichtlippe (3) derart abgestimmt ist, dass sich im Nasenflügel- oder Oberlippenbereich eine Artikulationsachse (X, A) ergibt

7. Dichtlippeneinrichtung nach wenigstens einem der Ansprüche 1 bis 6, **dadurch gekennzeichnet, dass** in dem; den Nasenflügeln oder der Oberlippe benachbarten Bereich der Dichtlippe (3) Zonen (4) höherer Tragfähigkeit ausgebildet sind.

8. Dichtlippeneinrichtung nach wenigstens einem der Ansprüche 1 bis 7, **dadurch gekennzeichnet, dass** die Zonen höherer Tragfähigkeit durch lokal verdickte Zonen der Dichtlippe (3) gebildet sind.

9. Dichtlippeneinrichtung nach wenigstens einem der Ansprüche 1 bis 8, **dadurch gekennzeichnet, dass** sich die lokal verdickten Zonen (4) über eine in die Dichtlippe (3) eingeformte Stützstruktur auf einer Maskenrahmenzone abstützen.

10. Dichtlippeneinrichtung nach wenigstens einem der Ansprüche 1 bis 9, **dadurch gekennzeichnet, dass** die Zonen (4) höherer Tragfähigkeit Pad-artig ausgebildet sind.

11. Dichtlippeneinrichtung nach wenigstens einem der Ansprüche 1 bis 10, **dadurch gekennzeichnet, dass** die Zonen (4) höherer Tragfähigkeit im Bereich der Gesichtskontaktzone jeweils eine im wesentlichen mondsichelförmige Gestalt aufweisen.

12. Dichtlippeneinrichtung nach wenigstens einem der Ansprüche 1 bis 11, **dadurch gekennzeichnet, dass** im Bereich der Oberlippe in einem zwischen den Zonen (4) höherer Tragfähigkeit liegenden Bereich eine Zone (c) hoher Nachgiebigkeit in und entgegen der Applikationsrichtung ausgebildet ist.

13. Dichtlippeneinrichtung nach wenigstens einem der Ansprüche 1 bis 12, **dadurch gekennzeichnet, dass** die Dichtlippeneinrichtung (1) an einem Maskenbasiskörper (12) angebracht ist.

14. Dichtlippeneinrichtung nach wenigstens einem der Ansprüche 1 bis 13, **dadurch gekennzeichnet, dass** der Maskenbasiskörper (12) durch eine Hartschale gebildet ist.

15. Dichtlippeneinrichtung nach wenigstens einem der Ansprüche 1 bis 14, **dadurch gekennzeichnet, dass** die Hartschale einen zum Stirnbereich weisenden Leitungsanschluss (60) aufweist.

16. Dichtlippeneinrichtung nach wenigstens einem der Ansprüche 1 bis 15, **dadurch gekennzeichnet, dass** eine Koppelungsstruktur vorgesehen ist, zur Koppelung der Dichtlippeneinrichtung (3) mit der Hartschale (12).

17. Dichtlippeneinrichtung nach wenigstens einem der Ansprüche 1 bis 16, **dadurch gekennzeichnet, dass** die Koppelungsstruktur seitens der Hartschale einen Umfangswulstabschnitt (12a) und seitens der Dichtlippeneinrichtung einen Rahmenabschnitt (8) mit einem komplementären Aufnahmefalz aufweist.

18. Dichtlippeneinrichtung nach wenigstens einem der Ansprüche 1 bis 17, **dadurch gekennzeichnet, dass** Mittel (22) vorgesehen sind zur Fixierung der Position der Dichtlippeneinrichtung (3) gegenüber der Hartschale in Umfangsrichtung.

19. Dichtlippeneinrichtung nach wenigstens einem der Ansprüche 1 bis 18, **dadurch gekennzeichnet, dass** der Dichtlippeneinrichtung (3) eine Vorspannung verliehen ist die durch elastische Verformung bei der Koppelung mit der Hartschale erreicht wird.

20. Dichtlippeneinrichtung nach wenigstens einem der Ansprüche 1 bis 19, **dadurch gekennzeichnet, dass** der Rahmenabschnitt (8) im wesentlichen in einer Ebene (f) verläuft.

21. Dichtlippeneinrichtung nach wenigstens einem der Ansprüche 1 bis 20, **dadurch gekennzeichnet, dass** der Rahmenabschnitt (8) eine im Bereich der Artikulationsachse (X,A) zur Zone hoher Tragfähigkeit (4) vordringenden Verlauf aufweist.

22. Dichtlippeneinrichtung nah wenigstens einem der Ansprüche 1 bis 21, **dadurch gekennzeichnet, dass** die Wanddicke der dünnen Zone der Dichtlippe (3) im Bereich von 0,65 bis 1,85 mm liegt.

23. Dichtlippeneinrichtung nach wenigstens einem der Ansprüche 1 bis 22, **dadurch gekennzeichnet, dass** die Wanddicke der Zone hoher Tragfähigkeit im Bereich von 0,80 bis 4mm liegt.

24. Dichtlippeneinrichtung nach wenigstens einem der Ansprüche 1 bis 23, **dadurch gekennzeichnet, dass** die Zone (4) hoher Tragfähigkeit eine andere Färbung aufweist als die dünnwandigere Zone.

25. Dichtlippeneinrichtung nach wenigstens einem der Ansprüche 1 bis 24, **dadurch gekennzeichnet, dass** die Zone (4) hoher Tragfähigkeit durch zwei vom unteren Eckbereich des Rahmenabschnittes aufragende und in die Dichtlippe als flache Schenkel ausfließende elastomere Abschnitte (5) gebildet ist.

26. Dichtlippeneinrichtung nach wenigstens einem der Ansprüche 1 bis 25, **dadurch gekennzeichnet, dass** die Dichtlippe (3) aus einem elastomeren Material insbesondere Silikonkautschuk gebildet ist.

27. Atemmaske mit einem Maskenbasiskörper und einer Dichtlippeneinrichtung nach einem der Ansprüche 1-26.

28. Atemmaske nach Anspruch 27, **dadurch gekennzeichnet, daß** die Hartschale an die Dichtlippeneinrichtung angespritzt ist.

## Claims

1. A sealing lip means for a breathing mask comprising a receiving opening for receiving at least the nose tip area of a mask user, a sealing lip (3) which is made of an elastomeric material, surrounds the receiving opening, crosses the nose bridge and comprises a contact zone which is intended to be placed on the face of a mask user, wherein the sealing lip (3) is arranged in such an elastically flexible manner that the sealing lip zone (a) which seals the area of the nose bridge has a higher flexibility than the sealing lip zone (b1, b2; c) which is adjacent to the nose wings and/or the upper lip of a mask user in the application position of the breathing mask, wherein the sealing lip (3) is suspended on a folded bellows structure (9) in a zone (a) that is intended for sealing the nose bridge region, **characterized in that** said folded bellows structure (9) has joint characteristics defined by different wall thicknesses.

2. The sealing lip means according to claim 1, **characterized in that** the folded bellows structure (9) forms a stop means.

3. The sealing lip means according to at least one of claims 1 to 2, **characterized in that** the folded bellows structure (9) comprises a plurality of folds.

4. The sealing lip means according to at least one of claims 1 to 3, **characterized in that** at least one fold extends from the nose bridge area to an area that neighbors the nose wings in the application position of the mask.

5. The sealing lip means according to at least one of claims 1 to 4, **characterized in that** at least one fold extends around the entire circumference of the sealing lip means.

6. The sealing lip means according to at least one of claims 1 to 5, **characterized in that** the flexibility of the sealing lip (3) with respect to the application direction (Z) is adjusted such that an articulation axis (X, A) is formed in the area of the nose wings or upper lip.

7. The sealing lip means according to at least one of claims 1 to 6, **characterized in that** zones (4) of increased carrying capacity are formed in the area of the sealing lip (3) that is adjacent to the nose wings or the upper lip.

8. The sealing lip means according to at least one of claims 1 to 7, **characterized in that** the zones of increased carrying capacity are formed by locally thickened zones of the sealing lip (3).

9. The sealing lip means according to at least one of claims 1 to 8, **characterized in that** the locally thickened zones (4) are supported on a mask frame zone by means of a supporting structure formed in the sealing lip (3).

10. The sealing lip means according to at least one of claims 1 to 9, **characterized in that** the zones (4) of increased carrying capacity are configured in a pad-like manner.

11. The sealing lip means according to at least one of claims 1 to 10, **characterized in that** the zones (4) of increased carrying capacity each have essentially the shape of a crescent moon in the area of the face contact zone.

12. The sealing lip means according to at least one of claims 1 to 11, **characterized in that** in the area of the upper lip a zone (c) of high flexibility in and contrary to the application direction is formed in an area lying between the zones (4) of increased carrying capacity.

13. The sealing lip means according to at least one of claims 1 to 12, **characterized in that** the sealing lip means (1) is attached to a mask base body (12).

14. The sealing lip means according to at least one of claims 1 to 13, **characterized in that** the mask base body (12) is formed by a hard shell.

15. The sealing lip means according to at least one of claims 1 to 14, **characterized in that** the hard shell comprises a conduit connection (60) facing towards the forehead area.

16. The sealing lip means according to at least one of claims 1 to 15, **characterized in that** a coupling structure is provided for coupling the sealing lip means (3) to the hard shell (12).

17. The sealing lip means according to at least one of claims 1 to 16, **characterized in that** on the hard shell the coupling structure comprises a circumferential bulge portion (12a) and on the sealing lip means a frame portion (8) with a complementary receiving fold.

18. The sealing lip means according to at least one of claims 1 to 17, **characterized in that** means (22) are provided for fixing the position of the sealing lip means (3) relative to the hard shell in the circumferential direction.

19. The sealing lip means according to at least one of claims 1 to 18, **characterized in that** the sealing lip means (3) is pre-stressed, the pre-stressing being caused by the elastic deformation which results when coupling the sealing lip means with the hard shell.

20. The sealing lip means according to at least one of claims 1 to 19, **characterized in that** the frame portion (8) extends essentially in a plane (f).

21. The sealing lip means according to at least one of claims 1 to 20, **characterized in that** the frame portion (8) has a course which extends in the area of the articulation axis (X, A) to the zone of high carrying capacity (4).

22. The sealing lip means according to at least one of claims 1 to 21, **characterized in that** the wall thickness of the thin zone of the sealing lip (3) lies in the range of 0.65 to 1.85 mm.

23. The sealing lip means according to at least one of claims 1 to 22, **characterized in that** the wall thickness of the zone of high carrying capacity lies in the range of 0.80 to 4 mm.

24. The sealing lip means according to at least one of claims 1 to 23, **characterized in that** the zone (4) of high carrying capacity has a color different from that of the thinwalled zone.

25. The sealing lip means according to at least one of claims 1 to 24, **characterized in that** the zone (4) of high carrying capacity is formed by two elastomeric portions (5) projecting from the lower edge area of the frame portion and tapering off in the sealing lip as flat legs.

26. The sealing lip means according to at least one of claims 1 to 25, **characterized in that** the sealing lip (3) is made of an elastomeric material, in particular silicone rubber.

27. A breathing mask comprising a mask base body and a sealing lip means according to any one of claims 1 to 26.

28. The breathing mask according to claim 27, **characterized in that** the hard shell is injection molded to the sealing lip means.

## Revendications

1. Dispositif à lèvre d'étanchéité pour un masque respiratoire, avec :
- une ouverture de logement pour recevoir au moins une région de pointe de nez d'un applicateur de masque,
- une lèvre d'étanchéité (3) fabriquée en un matériau élastomère faisant le pourtour de l'ouverture de logement et traversant le dos de nez,
qui présente une zone de pose, prévue pour une pose sur un visage d'un applicateur de masque,
la lèvre d'étanchéité (3) étant disposée de façon élastiquement déformable de sorte qu'il en résulte pour la zone de lèvre d'étanchéité (a), isolant de façon étanche la région du dos de nez, une plus grande déformabilité que pour la zone de lèvre d'étanchéité (b1, b2 ; c), qui est voisine des ailes du nez et/ou de la lèvre supérieure d'un applicateur du masque, lorsque le masque respiratoire est en position d'application,
la lèvre d'étanchéité (3) étant accrochée dans la région de la zone (a) prévue pour l'étanchéité de la région de dos de nez, à une structure à soufflet (9) réalisée localement, **caractérisé en ce que** la structure à soufflet présente une caractéristique d'articulation définie par des épaisseurs de paroi différentes.

2. Dispositif à lèvre d'étanchéité selon la revendication 1, **caractérisé en ce que** la structure à soufflet (9) forme un dispositif de butée.

3. Dispositif à lèvre d'étanchéité selon au moins l'une des revendications 1 à 2, **caractérisé en ce que** la structure à soufflet (9) comporte plusieurs enfoncements à plis.

4. Dispositif à lèvre d'étanchéité selon au moins l'une des revendications 1 à 3, **caractérisé en ce qu'**au moins un enfoncement à plis s'étend de la région de dos de nez jusqu'à une région qui est voisine des ailes du nez, lorsque le masque est en position d'utilisation.

5. Dispositif à lèvre d'étanchéité selon au moins l'une des revendications 1 à 4, **caractérisé en ce qu'**au moins un enfoncement à plis s'étend autour de l'ensemble de la périphérie du dispositif à lèvre d'étanchéité.

6. Dispositif à lèvre d'étanchéité selon au moins l'une des revendications 1 à 5, **caractérisé en ce que** la déformabilité, se référant à la direction d'application (Z) de la lèvre d'étanchéité (3), est adaptée de manière à obtenir un axe d'articulation (X, A) dans la région d'aile du nez ou de lèvre supérieure.

7. Dispositif à lèvre d'étanchéité selon au moins l'une des revendications 1 à 6, **caractérisé en ce que**, dans la région, voisine des ailes de nez ou de lèvre supérieure, de la lèvre d'étanchéité (3) sont réalisées des zones (4) à plus grande aptitude support.

8. Dispositif à lèvre d'étanchéité selon au moins l'une des revendications 1 à 7, **caractérisé en ce que** les zones à plus grande aptitude support sont formées par des zones, épaissies localement, de la lèvre d'étanchéité (3).

9. Dispositif à lèvre d'étanchéité selon au moins l'une des revendications 1 à 8, **caractérisé en ce que** les zones (4) épaissies localement prennent appui, par une structure d'appui formée dans la lèvre d'étanchéité (3), sur une zone de cadre de masque.

10. Dispositif à lèvre d'étanchéité selon au moins l'une des revendications 1 à 9, **caractérisé en ce que** les zones (4) à plus grande aptitude support sont réalisées à la façon de bourrelets.

11. Dispositif à lèvre d'étanchéité selon au moins l'une des revendications 1 à 10, **caractérisé en ce que** les zones (4) à plus grande aptitude support comportent dans la région de la zone de contact avec le visage, chacune, une forme sensiblement en croissant de lune.

12. Dispositif à lèvre d'étanchéité selon au moins l'une des revendications 1 à 11, **caractérisé en ce que**, dans la région de la lèvre supérieure, dans une région située entre les zones (4) à plus grande aptitude support, est réalisée une zone (c) à plus forte déformabilité, dans et à l'encontre de la direction d'application.

13. Dispositif à lèvre d'étanchéité selon au moins l'une des revendications 1 à 12, **caractérisé en ce que** le dispositif à lèvre d'étanchéité (1) est monté sur un corps de base de masque (12).

14. Dispositif à lèvre d'étanchéité selon au moins l'une des revendications 1 à 13, **caractérisé en ce que** le corps de base de masque (12) est formé par une coque dure.

15. Dispositif à lèvre d'étanchéité selon au moins l'une des revendications 1 à 14, **caractérisé en ce que** la coque dure comprend un raccordement de conduite (60) tourné vers la zone frontale.

16. Dispositif à lèvre d'étanchéité selon au moins l'une des revendications 1 à 15, **caractérisé en ce qu'**une structure de couplage est prévue, pour coupler le dispositif à lèvre d'étanchéité (3) à la coque dure (12).

17. Dispositif à lèvre d'étanchéité selon au moins l'une des revendications 1 à 16, **caractérisé en ce que** la structure de couplage présente, du côté de la coque dure, un tronçon à bourrelet périphérique (12a) et, du côté du dispositif à lèvre d'étanchéité, un tronçon de cadre (8) avec un pli de logement complémentaire.

18. Dispositif à lèvre d'étanchéité selon au moins l'une des revendications 1 à 17, **caractérisé en ce que** des moyens (22) sont prévus pour fixation de la position du dispositif à lèvre d'étanchéité (3) par rapport à la coque dure, dans la direction périphérique.

19. Dispositif à lèvre d'étanchéité selon au moins l'une des revendications 1 à 18, **caractérisé en ce que** le dispositif à lèvre d'étanchéité (3) se voit attribuer une précontrainte, obtenue par déformation élastique lors du couplage avec la coque dure.

20. Dispositif à lèvre d'étanchéité selon au moins l'une des revendications 1 à 19, **caractérisé en ce que** le tronçon de cadre (8) s'étend sensiblement dans un plan (f).

21. Dispositif à lèvre d'étanchéité selon au moins l'une des revendications 1 à 20, **caractérisé en ce que** le tronçon de cadre (8) comporte une allure pénétrant dans la région de l'axe d'articulation (X, A), vers la zone à plus grande aptitude support (4).

22. Dispositif à lèvre d'étanchéité selon au moins l'une des revendications 1 à 21, **caractérisé en ce que** l'épaisseur de paroi de la zone mince de la lèvre d'étanchéité (3) est comprise dans la fourchette de 0,65 à 1,85 mm.

23. Dispositif à lèvre d'étanchéité selon au moins l'une des revendications 1 à 22, **caractérisé en ce que** l'épaisseur de paroi de la zone à plus grande aptitude support est comprise dans la fourchette de 0,80 à 4 mm.

24. Dispositif à lèvre d'étanchéité selon au moins l'une des revendications 1 à 23, **caractérisé en ce que** la zone (4) à plus grande aptitude support comporte une autre coloration que la zone à paroi plus mince.

25. Dispositif à lèvre d'étanchéité selon au moins l'une des revendications 1 à 24, **caractérisé en ce que** la zone (4) à plus grande aptitude support est formée par deux tronçons (5) en élastomère, faisant saillie depuis la zone d'angle inférieure du tronçon de cadre et évoluant en la lèvre d'étanchéité sous forme de branche plate.

26. Dispositif à lèvre d'étanchéité selon au moins l'une des revendications 1 à 25, **caractérisé en ce que** la lèvre d'étanchéité (3) est formée d'un matériau élastomère, en particulier de caoutchouc au silicone.

27. Masque respiratoire, comportant un corps de base de masque et un dispositif à lèvre d'étanchéité selon l'une des revendications 1 à 26.

28. Masque respiratoire selon la revendication 27, **caractérisé en ce que** la coque dure est rapportée par injection sur le dispositif à lèvre d'étanchéité.
